# FASCICULE DE BREVET EUROPEEN

(11) **EP 1 698 326 B1**
(45) Date de publication et mention de la délivrance du brevet: **04.08.2010**
(21) Numéro de dépôt: 06000353.0
(22) Date de dépôt: 10.01.2006
(51) Int. Cl.: A61K 8/73, A61K 8/34, A61Q 5/02, A61Q 5/12

(54) **Composition cosmétique comprenant un tensioactif cationique, un polymère cationique, un composé solide et un amidon et procédé de traitement cosmétique**
Kosmetische Zusammensetzung enthaltend ein kationisches Tensid, ein kationisches Polymer, einen Feststoff und Stärke, und kosmetisches Behandlungsverfahren
Cosmetic composition comprising a cationic surfactant, a cationic polymer, a solid compound and a starch, and cosmetic treatment process

(30) Priorité: 11.02.2005 FR 0501414
(43) Date de publication de la demande: 06.09.2006
(73) Titulaire: L'Oréal, 75008 Paris (FR)
(72) Inventeur: Lazzeri, Pascale, 92300 Levallois Perret (FR); Chesneau, Laurent, 92300 Levallois Perret (FR); Mahe, Véronique, 78740 Vaux sur Seine (FR)
(74) Mandataire: Le Blainvaux Bellegarde, Françoise

(56) Documents cités:
- EP-A- 0 968 703
- DE-A1- 10 216 500
- US-A- 5 013 763
- US-A- 5 756 077
- US-A1- 2001 031 270
- US-A1- 2003 099 692

## Description

La présente invention est relative à une composition cosmétique notamment de conditionnement des cheveux comprenant un tensioactif cationique, au moins un amidon modifié, au moins un polymère cationique non siliconé de charge cationique élevée et au moins un composé solide non polymérique non ionique particulier et à un procédé de traitement cosmétique des matières kératiniques en particulier les cheveux.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà proposé pour le traitement des matières kératiniques et en particulier des cheveux, des compositions cosmétiques comprenant tensioactifs cationiques et des polysaccharides épaississants tels que notamment l'amidon ou les celluloses.

De telles compositions présentent cependant des inconvénients tels que des problèmes de rinçabilité, des problèmes de stabilité, des difficultés de répartition sur les matières kératiniques ainsi que des propriétés cosmétiques insuffisantes.

On a déjà préconisé dans les compositions pour le lavage ou le soin des matières kératiniques telles que les cheveux l'utilisation de polymères cationiques, de silicones cationiques ou de tensioactifs cationiques pour faciliter le démêlage des cheveux et pour leur communiquer douceur et souplesse. L'usage des polymères cationiques ou des cations dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage.

DE 102 16 500 A1 (BEIERSDORF AG) décrit (ex. 1) une composition cosmétique comprenant dans un milieu cosmétiquement acceptable, un tensioactif cationique (cetrimonium bromide), au moins un amidon (hydroxypropyl starch phosphate ester), au moins un polymère cationique non siliconé (polyquaternium-10) et au moins un composé solide non polymérique non ionique présentant un point de fusion supérieur ou égal à 35°C (alcool cetearylique).

De plus les soins utilisés pour les cheveux très sensibilisés peuvent être insuffisant pour traiter les pointes qui sont le plus souvent très abîmées.

En résumé, il s'avère que les compositions cosmétiques actuelles de conditionnement, ne donnent pas complètement satisfaction. Ainsi, on cherche à obtenir des compositions cosmétiques ayant de très bonnes propriétés cosmétiques en particulier sur des cheveux très sensibilisés.

La demanderesse a maintenant découvert que l'association d'un tensioactif cationique, d'un amidon de préférence modifié, d'un polymère cationique non siliconé de charge cationique particulière et d'un composé solide non polymérique non ionique particulier permet de remédier à ces inconvénients.

Les cheveux traités avec cette composition sont lisses, se démêlent facilement, sont brillants, souples, individualisés et ont un toucher doux et sans résidus. Les cheveux ont un aspect naturel et non chargé. Le lissage est homogène des racines aux pointes. Les pointes présentent moins de fourches.

De plus, ces effets sont rémanents au cours du temps.

Ainsi, selon la présente invention, il est maintenant proposé de nouvelles compositions cosmétiques, comprenant, dans un milieu aqueux cosmétiquement acceptable, au moins un tensioactif cationique, au moins un amidon de préférence modifié, au moins un polymère cationique non siliconé présentant une densité de charge cationique supérieure ou égale à 5 meq/g et au moins un composé solide non polymérique non ionique de point de fusion supérieur ou égal à 35°C et/ou ayant une viscosité à la température de 40°C et sous un taux de cisaillement de 1s⁻¹, supérieure ou égale à 1 Pa.s.

Un autre objet de l'invention consiste en un procédé de traitement cosmétique des matières kératiniques, en particulier les cheveux mettant en oeuvre la composition susvisée.

L'invention a encore pour objet l'utilisation de ladite composition comme après-shampoing.

D'autres objets, caractéristiques, aspects et avantages de l'invention apparaîtront encore plus clairement à la lecture de la description et des divers exemples qui suivent.

Par cheveux sensibilisés, on comprend selon la présente invention, des cheveux ayant subi des agressions extérieures physiques (lumière, chaleur, ondes...), des agressions mécaniques (brushing, peignages ou brossages répétés...) ou des agressions chimiques (coloration d'oxydation, décoloration, permanente, défrisage...). Parmi ces agressions, on notera le caractère particulièrement délétère des agressions chimiques. Les compositions selon l'invention sont particulièrement efficaces sur les cheveux sensibilisés par des agressions chimiques.

Par « au moins un », on comprendra « un ou plusieurs », C'est-à-dire un, deux, trois ou plus.

Par "milieu cosmétiquement acceptable", on entend un milieu compatible avec toutes les matières kératiniques telles que la peau, les cheveux, les ongles, les cils, les sourcils, les lèvres et toute autre zone du corps et du visage.

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

Selon l'invention, les tensioactifs cationiques sont de préférence non polymériques.

A titre de sels d'ammonium quaternaire, on peut notamment citer, par exemple :
- ceux qui présentent la formule générale (V) suivante : 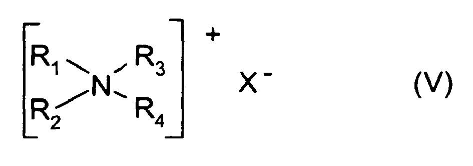 dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle en C1-C30, , alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline comme, par exemple, ceux de formule (VI) suivante : 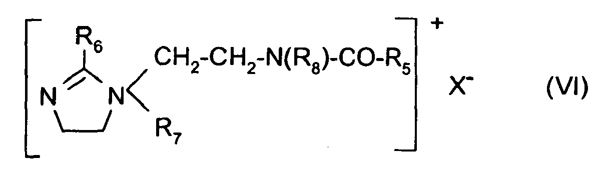 dans laquelle R₅ représente un radical alcényle ou alkyle comportant de 8 à 30 atomes de carbone par exemple dérivés des acides gras du suif ou du coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 2002), le Quaternium-87 (CTFA 2002) ou le Quaternium-83 (CTFA 2002) commercialisés sous les dénominations "VARISOFT® " W575PG par la société GOLDSCHMIDT,
- les sels de diammonium quaternaire de formule (VII) : 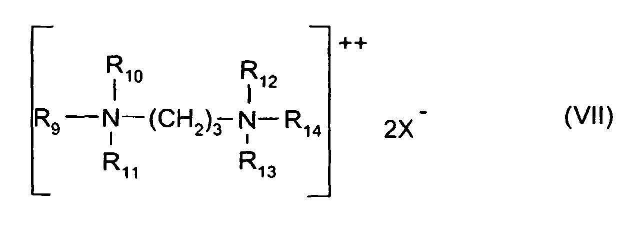 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X⁻ est un anion choisi dans le groupe des halogénures, acétates, phosphates, nitrates, éthylsulfates et méthylsulfates. De tels sels de diammonium quaternaire comprennent notamment le dichlorure de propanesuif diammonium ;
- les sels d'ammonium quaternaire comprenant au moins une fonction ester, tels que ceux de formule (VIII) suivante : 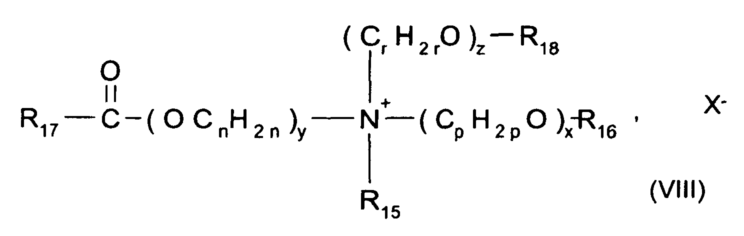 dans laquelle :
   R₁₅ est choisi parmi les radicaux alkyles en C₁-C₆ et les radicaux hydroxyalkyles ou dihydroxyalkyles en C₁-C₆ ;
   R₁₆ est choisi parmi :
      - le radical 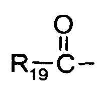
      - les radicaux R₂₀ hydrocarbonés en C₁-C₂₂, linéaires ou ramifiés, saturés ou insaturés,
      - l'atome d'hydrogène,
   R₁₇ est choisi parmi :
      - le radical 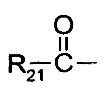
      - les radicaux R₂₂ hydrocarbonés en C₁-C₆, linéaires ou ramifiés, saturés ou insaturés,
      - l'atome d'hydrogène,
         R₁₇, R₁₉ et R₂₁, identiques ou différents, sont choisis parmi les radicaux hydrocarbonés en C₇-C₂₁, linéaires ou ramifiés, saturés ou insaturés ;
         r, n et p, identiques ou différents, sont des entiers valant de 2 à 6 ;
         y est un entier valant de 1 à 10 ;
         x et z, identiques ou différents, sont des entiers valant de 0 à 10 ;
         X⁻ est un anion simple ou complexe, organique ou inorganique ;
         sous réserve que la somme x + y + z vaut de 1 à 15, que lorsque x vaut 0 alors R₁₆ désigne R₂₀ et que lorsque z vaut 0 alors R₁₈ désigne R₂₂.
         Les radicaux alkyles R₁₅ peuvent être linéaires ou ramifiés, et plus particulièrement linéaires.
         De préférence, R₁₅ désigne un radical méthyle, éthyle, hydroxyéthyle ou dihydroxypropyle, et plus particulièrement un radical méthyle ou éthyle.
         Avantageusement, la somme x + y + z vaut de 1 à 10.
         Lorsque R₁₆ est un radical R₂₀ hydrocarboné, il peut être long et avoir de 12 à 22 atomes de carbone, ou court et avoir de 1 à 3 atomes de carbone.
         Lorsque R₁₈ est un radical R₂₂ hydrocarboné, il a de préférence 1 à 3 atomes de carbone.
         Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.
         De préférence, x et z, identiques ou
         différents, valent 0 ou 1.
         Avantageusement, y est égal à 1.
         De préférence, r, n et p, identiques ou différents, valent 2 ou 3, et encore plus particulièrement sont égaux à 2.
         L'anion X- est de préférence un halogénure (chlorure, bromure ou iodure) ou un alkyl(C1-C4)sulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.
         L'anion X⁻ est encore plus particulièrement le chlorure ou le méthylsulfate.
         On utilise plus particulièrement dans la composition selon l'invention, les sels d'ammonium de formule (IV) dans laquelle :
      - R₁₅ désigne un radical méthyle ou éthyle,
      - x et y sont égaux à 1 ;
      - z est égal à 0 ou 1 ;
      - r, n et pt sont égaux à 2 ;
      - R₁₆ est choisi parmi :
      - le radical 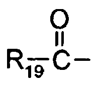
      - les radicaux méthyle, éthyle ou hydrocarbonés en C₁₄-C₂₂,
      - l'atome d'hydrogène ;
      - R₁₈ est choisi parmi :
      - le radical 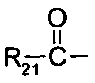
      - l'atome d'hydrogène ;
      - R₁₇, R₁₉ et R₂₁, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.
         Avantageusement, les radicaux hydrocarbonés sont linéaires.

On peut citer par exemple les composés de formule (VIII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société COGNIS, STEPANQUAT® par la société STEPAN, NOXAMIUM® par la société CECA, REWOQUAT® WE 18 par la société REWO-GOLDSCHMIDT.

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange comprenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium comprenant au moins une fonction ester décrits dans les brevets US-A-4874554 et US-A-4137180.

Parmi les sels d'ammonium quaternaire mentionnés ci-dessus, on préfère utiliser ceux répondant à la formule (V). On peut notamment citer d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium correspondant au QUATERNIUM-70 (CTFA 2002) commercialisé sous la dénomination CERAPHYL® 70 par la société ISP.

Les tensioactifs cationiques particulièrement préférés dans la composition de l'invention sont choisis parmi les sels d'ammonium quaternaire, et en particulier parmi le chlorure de béhényltriméthylammonium, le chlorure de cétyltriméthylammonium, le quaternium-83, le quaternium-87, le chlorure de béhénylamidopropyl 2,3-dihydroxypropyl diméthyl ammonium et le chlorure de palmitylamidopropyltriméthylammonium.

La composition selon l'invention comprend de préférence le ou les tensioactifs cationiques en une quantité allant de 0,05 à 10 % en poids, de préférence de 0,1 à 8 % en poids par rapport au poids total de la composition et plus particulièrement de 0,2 à 5% en poids.

La composition cosmétique selon l'invention comprend un ou plusieurs polymères cationiques dont la densité de charge cationique est supérieure ou égale à 5 milliéquivalents par gramme (meq/g), de préférence allant de 5 à 20 méq/g.

La densité de charge cationique d'un polymère correspond au nombre de moles de charges cationiques par unité de masse de polymère dans les conditions où celui-ci est totalement ionisé. Elle peut être déterminée par calcul si on connaît la structure du polymère, c'est-à-dire la structure des monomères constituant le polymère et leur proportion molaire ou pondérale. Elle peut être aussi déterminée expérimentalement par la méthode Kjeldahl.

Les polymères cationiques ayant une densité de charge cationique supérieure ou égale à 5 méq/g, utilisables conformément à la présente invention, peuvent être choisis parmi tous ceux déjà connus en soi comme améliorant les propriétés cosmétiques des cheveux traités par des compositions, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière générale, au sens de la présente invention, l'expression "polymère cationique" désigne tout polymère comprenant des groupes cationiques et/ou des groupes ionisables en groupes cationiques.

Les polymères cationiques sont choisis parmi ceux qui comprennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les copolymères vinylpyrrolidone/(méth)acrylate de dialkylamino-alkyle quaternisés ou non,
(2) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361 ;
(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alkylés ou s'ils comportent une ou plusieurs fonctions amines tertiaires, quaternisés. De tels polymères sont notamment décrits dans les brevets français 2 252 840 et 2 368 508 ;
(4) les dérivés de polyaminoamides résultant de la condensation de polyalkylènes-polyamines avec des acides polycarboxyliques suivie d'une alkylation par des agents bifonctionnels. On peut citer, par exemple, les polymères acide adipique/diakylaminohydroxyalkyl-dialkylène-triamine dans lesquels le groupe alkyle comporte de 1 à 4 atomes de carbone et désigne de préférence méthyle, éthyle, propyle. De tels polymères sont notamment décrits dans le brevet français 1 583 363.
(5) les polymères obtenus par réaction d'une polyalkylène-polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.
(6) les cyclopolymères d'alkyldiallylamine ou de dialkyldiallyl-ammonium tels que les homopolymères ou copolymères comportant des motifs répondant aux formules (VI) ou (VI') : 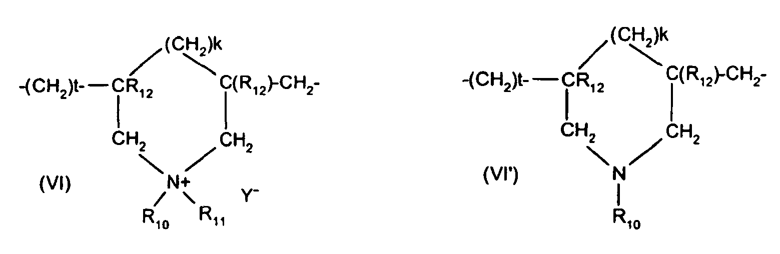 formules dans lesquelles k et t sont égaux à 0 ou 1, la somme k + t étant égale à 1 ; R₁₂ désigne un atome d'hydrogène ou un groupe méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur, ou R₁₀ et R₁₁ peuvent désigner conjointement avec l'atome d'azote auquel ils sont rattachés, des groupement hétérocycliques, tels que pipéridinyle ou morpholinyle ; Y est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.
   On peut citer, par exemple, l'homopolymère de chlorure de diallyldiméthylammonium commercialisé sous la dénomination "MERQUAT® 100" par la société ONDEO-NALCO et les copolymères de chlorure de diallyldiméthylammonium et d'acrylamide.
(7) le polycondensat de diammonium quaternaire comprenant des motifs récurrents répondant à la formule : 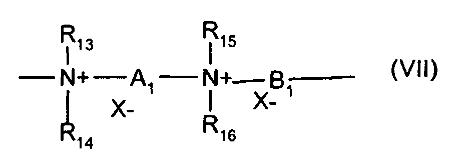 formule (VII) dans laquelle :
   R₁₃, R₁₄, R₁₅ et R₁₆, identiques ou différents, représentent des groupes aliphatiques, alicycliques, ou aryle aliphatiques comprenant de 1 à 20 atomes de carbone ou des groupes hydroxyalkyle aliphatiques inférieurs, ou bien R₁₃, R₁₄, R₁₅ et R₁₆, ensemble ou séparément, constituent avec les atomes d'azote auxquels ils sont rattachés des hétérocycles comprenant éventuellement un second hétéroatome autre que l'azote, ou bien R₁₃, R₁₄, R₁₅ et R₁₆ représentent un groupe alkyle en C₁-C₆, linéaire ou ramifié, substitué par un groupement nitrile, ester, acyle, amide ou -CO-O-R₁₇-D ou -CO-NH-R₁₇-D où R₁₇ est un groupe alkylène et D un groupement ammonium quaternaire ;
   A₁ et B₁ représentent des groupements polyméthyléniques comprenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique ;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement -(CH₂)ₙ-CO-D-OC-(CH₂)ₙ- dans lequel D désigne :
      a) un reste de glycol de formule : -O-Z-O-, où Z désigne un groupe hydrocarboné linéaire ou ramifié ou un groupement répondant à l'une des formules suivantes :

         -(CH₂-CH₂-O)x-CH₂-CH₂-

         -[CH₂-CH(CH3)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un groupe hydrocarboné linéaire ou ramifié, ou bien le groupe bivalent

         -CH₂-CH₂-S-S-CH₂-CH₂- ;
      d) un groupement uréylène de formule : -NH-CO-NH- ;
      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets français numéros 2 320 330, 2 270 846, 2 316 271, 2 336 434 et 2 413 907 et les brevets US numéros 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4.026.945 et 4 027 020.
      On peut utiliser plus particulièrement les polymères qui sont constitués de motifs récurrents répondant à la formule : 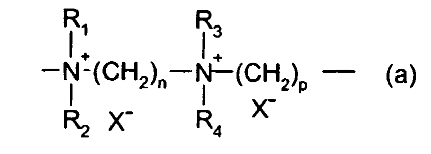 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
      Un composé de formule (a) particulièrement préféré est celui pour lequel R₁, R₂, R₃ et R₄ représentent un groupe méthyle et n = 3, p= 6 et X = CI, dénommé "Hexadimethrine chloride" selon la nomenclature INCI (CTFA).
(8) les polycondensats de polyammonium quaternaires constitués de motifs de formule (VIII): 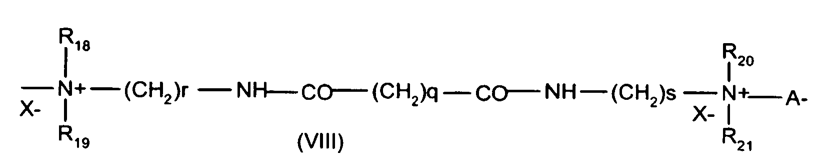 formule dans laquelle :
   R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH,
   où p est égal à 0 ou à un nombre entier allant de 1 à 6, sous réserve que R₁₈, R₁₉, R₂₀ et R₂₁ ne représentent pas simultanément un atome d'hydrogène,
   r et s, identiques ou différents, sont des nombres entiers allant de 1 à 6,
   q est égal à 0 ou à un nombre entier allant de 1 à 34,
   X désigne un atome d'halogène,
   A désigne un groupe dérivé d'un dihalogénure ou représente de préférence -CH₂-CH₂-O-CH₂-CH₂-.
      De tels composés sont notamment décrits dans la demande de brevet EP-A-122 324.
      On peut, par exemple, citer parmi ceux-ci, les produits "Mirapol® A 15", "Mirapol® AD1 ", "Mirapol® AZ1" et "Mirapol® 175" vendus par la société Miranol.
(9) les homopolymères ou copolymères dérivés des acides acrylique ou méthacrylique et comportant des motifs : 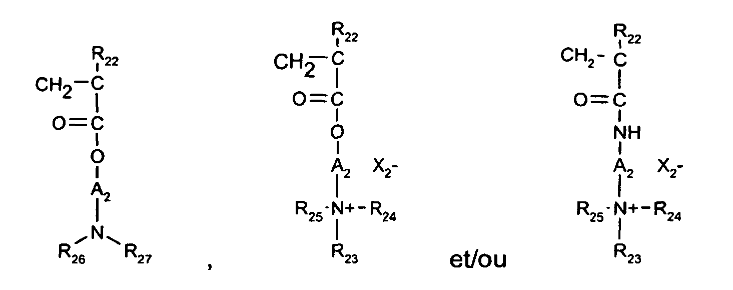 dans lesquels les groupements R₂₂ désignent indépendamment H ou CH₃,
   les groupements A₂ désignent indépendamment un groupe alkyle linéaire ou ramifié de 1 à 6 atomes de carbone, ou un groupe hydroxyalkyle de 1 à 4 atomes de carbone,
   les groupements R₂₃, R₂₄, R₂₅, identiques ou différents, désignant indépendamment un groupe alkyle de 1 à 18 atomes de carbone, ou un groupe benzyle,
   les groupements R₂₆ et R₂₇ représentent un atome d'hydrogène ou un groupement alkyle de 1 à 6 atomes de carbone,
   X₂⁻ désigne un anion, par exemple méthosulfate ou halogénure tel que chlorure ou bromure.
   Le ou les comonomères utilisables dans la préparation des copolymères correspondants appartiennent à la famille des acrylamides, méthacrylamides, diacétone-acrylamides, acrylamides et méthacrylamides substitués à l'azote par des alkyle inférieurs, des esters d'alkyle, des acides acrylique ou méthacrylique, la vinylpyrrolidone ou des esters vinyliques.
(10) Les polymères quaternaires de vinylpyrrolidone et de vinylimidazole.
(11) Les polymères réticulés de sels de méthacryloyloxyalkyl(C₁-C₄) trialkyl(C₁-C₄)ammonium tels que les polymères obtenus par homopolymérisation du méthacrylate de diméthylaminoéthyle quaternisé par le chlorure de méthyle, ou par copolymérisation de l'acrylamide avec le méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bis-acrylamide.

D'autres polymères cationiques utilisables dans le cadre de l'invention sont des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les homopolymères et copolymères d'halogénure de dialkyldiallylammonium, les polyéthylène-imines, les polycondensats à motifs récurrents diammonium ou polyammonium quaternaire.

Les polymères cationiques décrits ci-dessus sont présents de préférence en une quantité allant de 0,01 à 10 % en poids, mieux encore de 0,05 à 5 % en poids, et encore plus préférentiellement de 0,1 à 3 % en poids par rapport au poids total de la composition.

Les amidons utilisables dans la présente invention sont plus particulièrement des macromolécules sous forme de polymères constitués de motifs élémentaires qui sont des unités anhydroglucose. Le nombre de ces motifs et leur assemblage permettent de distinguer l'amylose (polymère linéaire) et l'amylopectine (polymère ramifié). Les proportions relatives d'amylose et d'amylopectine, ainsi que leur degré de polymérisation, varient en fonction de l'origine botanique des amidons.

Les molécules d'amidons utilisés dans la présente invention peuvent avoir comme origine botanique les céréales ou encore les tubercules. Ainsi, les amidons sont par exemple choisis parmi les amidons de maïs, de riz, de manioc, d'orge, de pomme de terre, de blé, de sorgho, de pois.

Les amidons utilisés dans la composition de l'invention sont de préférence modifiés. Les amidons peuvent être modifiés par voie chimique ou physique : notamment par une ou plusieurs des réactions suivantes : prégélatinisation, oxydation, réticulation, estérification, éthérification, amidification, traitements thermiques.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymèrisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glyceryl et/ou phosphate) ;
- estérification en milieu alcalin pour le greffage de groupes fonctionnels, notamment acyl en en C1-C6 (acétyl), hydroxyalkylés en C1-C6 (hydroxyéthyl, hydroxypropyl), carboxyméthyl, octénylsuccinique.

On peut notamment obtenir par réticulation avec des composés phophorés des phosphates de monoamidon ( du type Am-O-PO-(OX)₂), des phosphates de diamidon ( du type Am-O-PO-(OX)-O-Am) ou même de triamidon (du type Am-O-PO- (O-Am)₂) ou leurs mélanges.

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Les composés phosphorés peuvent être par exemple du tripolyphosphate de sodium, de l'orthophosphate de sodium, de l'oxychlorure de phosphore ou du trimétaphosphate de sodium.

On utilisera préférentiellement des phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs gélatinisé).

Un amidon préféré est un amidon ayant subi au moins une modification voie chimique telle qu'au moins une estérification.

Selon l'invention, on peut aussi utiliser des amidons amphotères, ces amidons amphotères comprennent un ou plusieurs groupements anioniques et un ou plusieurs groupements cationiques. Les groupements anioniques et cationiques peuvent être liés au même site réactif de la molécule d'amidon ou à des sites réactifs différents; de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les amidons amphotères sont notamment choisis parmi les composés de formules suivantes : 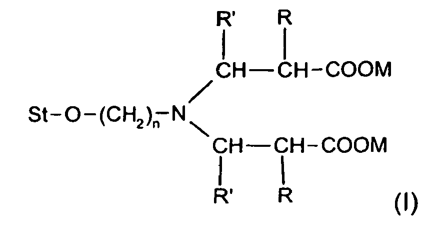 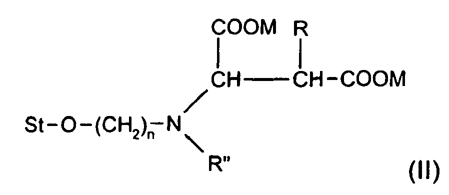 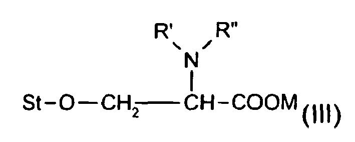 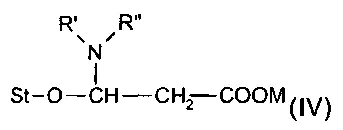 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, Li, NH₄ , un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460 qui sont inclus à titre de référence.

Les molécules d'amidons peuvent être issues de toutes les sources végétales d'amidon telles que notamment le maïs, la pomme de terre, l'avoine, le riz, le tapioca, le sorgho, l'orge ou le blé. On peut également utiliser les hydrolysats des amidons cités ci-dessus. L'amidon est de préférence issu de la pomme de terre.

On utilise particulièrement les amidons de formules (I) ou (II). On utilise plus particulièrement les amidons modifiés par de l'acide 2-chloroéthyl aminodipropionique, c'est à dire les amidons de formule (I) ou (II) dans lesquelles R, R', R" et M représentent un atome d'hydrogène et n est égal à 2. L'amidon amphotère préféré est un chloroéthylamidodipropionate d'amidon.

Selon l'invention, le ou les amidons de préférence modifiés peuvent représenter de 0,1 % à 20 % en poids, de préférence de 0,5 % à 15% en poids et plus particulièrement de 1 à 10% en poids par rapport au poids total de la composition finale.

Les composés solides selon l'invention peuvent être cristallisés, amorphes ou pateux.

Le point de fusions va de préférence de 35 à 250°C et plus particulièrement de 40 à 150°C.

Ces solides ont une viscosité à la température de 40°C et sous un taux de cisaillement de 1s⁻¹, allant de 1 Pa.s à 1000000 Pa.s et de préférence de 10 à 1000 Pa.s.

Les mesures de viscosités peuvent réalisées à une température d'environ 40°C, sur un Carri-Med CSL2-500.

Les points de fusion peuvent être mesurés par DSC ou sur un banc Kofler. Le point de fusion peut être mesuré par analyse calorimétrique différentielle (DSC) avec une vitesse de montée en température de 10 °C/min. Le point de fusion est alors la température correspondant au sommet du pic endotherme de fusion obtenu lors de la mesure.

Les composés solides non polymériques non ioniques présentant un point de fusion supérieur ou égal à 35 °c sont notamment choisis parmi les alcools gras oxyéthylénés ou non, les esters gras, les cires minérales et les cires organiques différentes des esters gras et des alcools gras et leurs mélanges.

Les alcools gras selon l'invention sont de préférence linéaires et saturés, et comportent de 12 à 40 atomes de carbone.

Les alcools gras présentent de préférence la structure R-OH, dans laquelle R désigne de préférence un groupement alkyle en C12-C24. R peut être substitué par un ou plusieurs groupements hydroxy.

A titre d'exemple on peut citer l'alcool myristique l'alcool cétylique, l'alcool stéarylique, l'alcool béhénylique et leurs mélanges.

L'alcool gras peut représenter un mélange d'alcools gras, ce qui signifie que dans un produit commercial peuvent coexister plusieurs espèces d'alcools gras, sous forme d'un mélange.

De préférence, les alcools gras de l'invention sont non oxyalkylénés et/ou non glycérolés. Ces alcools gras peuvent être des constituants des cires animales ou végétales.

Les esters gras sont des esters d'acides gras c'est-à-dire des esters d'un acide carboxylique comportant au moins 10 atomes de carbone et d'un monoalcool ou d'un polyol. Les esters gras selon l'invention peuvent être des mono, des di ou des triesters.

Les acides carboxyliques ont de préférence de 10 à 30 atomes de carbone et plus particulièrement de 12 à 24 atomes de carbone. Les alcool ont de préférence de 10 à 30 atomes de carbone et plus particulièrement de 12 à 24 atomes de carbone. De préférence, les esters gras solides selon l'invention sont des esters d'acide gras monocarboxylique comportant au moins 10 atomes de carbone et d'un monoalcool comportant au moins 10 atomes de carbone.

A titre d'esters selon l'invention, on peut citer le myristate de cétyle, le myristate de myristyle.

Les esters gras peuvent être des constituants des cires animales ou végétales.

Une cire, au sens de la présente invention, est un composé lipophile, solide à température ambiante (environ 25°C), à changement d'état solide/liquide réversible, ayant une température de fusion supérieure à environ 40°C et pouvant aller jusqu'à 200°C, et présentant à l'état solide une organisation cristalline anisotrope. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires utilisables dans la présente invention, on peut citer les cires d'origine animale telles que la cire d'abeille, le spermaceti, la cire de lanoline et les dérivés de lanoline ; les cires végétales telles que la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines, les ozokérite, la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, et leurs mélanges. Comme cires organiques, on peut aussi citer les cires comprenant des fonctions amides et en particulier les céramides naturelles ou synthétiques.

Sur la définition des cires, on peut citer par exemple P.D. Dorgan, Drug and Cosmetic Industry, Decembre 1983, pp. 30-33.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82.

Le ou les composés solides peuvent être présents dans la composition à une teneur allant de 0,1 à 15%, de préférence de 0,5 à 10%, encore de préférence de 1 à 8% en poids du poids total de la composition.

La composition selon l'invention peut contenir éventuellement d'autres tensioactifs que les tensioactifs cationiques.

Les tensioactifs peuvent être présents en une quantité allant de 0,1% à 10% en poids environ, de préférence de 0,5% à 8% et encore plus préférentiellement de 1 % à 5%, par rapport au poids total de la composition.

Les tensioactifs additionnels sont de préférence choisis parmi les tensioactifs non ioniques.

Les agents tensioactifs non-ioniques sont des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés, ou les acides gras polyéthoxylés, polypropoxylés ou polyglycérolés, tous ces composés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine.

Les compositions selon l'invention sont de préférence des compositions non lavantes (non détergentes), elles comprennent de préférence moins de 4% en poids de tensioactifs anioniques et plus particulièrement moins de 1% en poids par rapport au poids total de la composition.

La composition selon l'invention peut comprendre en outre au moins un agent conditionneur additionnel choisi parmi les silicones, les polymères cationiques différents des polymères cationiques selon l'invention, les esters gras carboxyliques différents de ceux de l'invention, les huiles végétales, les huiles minérales, les huiles de synthèse telles que les poly(alpha-oléfines), et leurs mélanges.

Les silicones utilisables conformément à l'invention peuvent être solubles ou insolubles dans la composition, et elles peuvent être en particulier des polyorganosiloxanes insolubles dans la composition de l'invention. Elles peuvent se présenter sous forme d'huiles, de cires, de résines ou de gommes. Elles peuvent être utilisées pures ou en émulsion, en dispersion ou en microémulsion.

Les organopolysiloxanes sont définis plus en détail dans l'ouvrage de Walter NOLL "Chemistry and Technology of Silicones" (1968) Academic Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i)les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 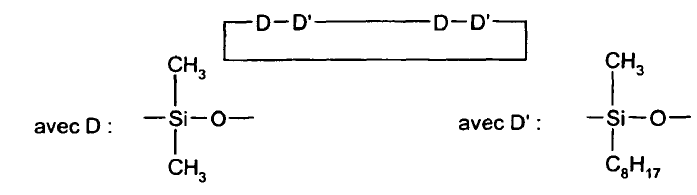 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".
   Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels ainsi que leurs mélanges.
   Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.
   Parmi les silicones organomodifiées, on peut citer les polyorganosiloxanes comportant :
   - des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C₁₂)-méthicone-copolyol commercialisée par la société DOW CORNING sous la dénomination Q2 5200 ;
   - des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
   - des groupements thiols, comme les produits commercialisés sous les dénominations "GP 72 A" et "GP 71" de GENESEE ;
   - des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
   - des groupements hydroxylés, comme les polyorganosiloxanes à fonction hydroxyalkyle décrits dans la demande de brevet français FR-A-85 16334 ;
   - des groupements acyloxyalkyle tels que, par exemple, les polyorganosiloxanes décrits dans le brevet US-A-4957732 ;
   - des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701^{E} de la société SHIN-ETSU ; 2-hydroxyalkylsulfonate ; 2-hydroxyalkylthiosulfate tels que les produits commercialisés par la société GOLDSCHMIDT sous les dénominations "ABIL® S201" et "ABIL® S255" ;
   - des groupements hydroxyacylamino, comme les polyorgano-siloxanes décrits dans la demande EP 342 834. On peut citer, par exemple, le produit Q2-8413 de la société DOW CORNING.
   A titre d'exemples de silicones, on utilise de préférence les polydiméthylsiloxanes, les polyalkylarylsiloxanes, les polydiméthylsiloxanes à groupements aminés ou alcoxylés.

La composition selon l'invention peut également comprendre un ou plusieurs esters liquides d'acide carboxylique, tels que par exemple, l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le lactate de 2-octyldodécyle, le néopentanoate d'isostéaryle, le néopentanoate de tridécyle, le néopentanoate d'isocétyle et le néopentanoate d'isoarachidyle et leurs mélanges.

La composition selon l'invention peut encore comprendre une ou plusieurs huiles végétales telles que l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum et leurs mélanges.

Comme huiles minérales, on peut notamment citer l'huile de paraffine et l'huile de vaseline.

Les agents conditionneurs additionnels sont contenus de préférence dans la composition selon l'invention en une quantité allant de 0,01 % à 20 % en poids, mieux encore allant de 0,1 % à 10 % en poids et plus particulièrement allant de 0,3 % à 5 % en poids par rapport au poids total de la composition.

Le milieu cosmétiquement acceptable est de préférence aqueux et peut être comprendre de l'eau ou un mélange d'eau et d'un solvant cosmétiquement acceptable tel qu'un alcool inférieur en C₁-C₄, par exemple l'éthanol, l'isopropanol, le tertio-butanol, le n-butanol ; les polyols comme le propylèneglycol, la glycérine ; les éthers de polyols ; les alcanes en C₅-C₁₀ ; et leurs mélanges.. Les solvants sont de préférence choisis parmi la glycérine et le propylène glycol.

Le milieu cosmétiquement acceptable notamment aqueux représente de préférence de 30 à 98% en poids par rapport au poids total de la composition.

Les solvants sont de préférence présents dans des concentrations allant de 0,5 à 30% en poids par rapport au poids total de la composition.

Le pH des compositions de l'invention est compris de préférence de 2 à 8, de préférence de 3 à 7.

Les compositions selon l'invention peuvent également contenir des additifs classiques bien connus dans la technique, tels que des polymères anioniques, non ioniques ou amphotères, des épaississants non polymériques comme des acides ou des électrolytes, des opacifiants, des nacrants, des vitamines, des provitamines telles que le panthénol, des parfums, des colorants, des particules organiques ou minérales, des conservateurs, des agents de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions de l'invention peuvent se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Elles peuvent être utilisées, par exemple, comme après-shampoings, soins, masques de soin profond, lotions ou crèmes de traitement du cuir chevelu. Ces compositions peuvent être rincées ou non rincées.

Selon un mode de réalisation préféré de l'invention, la composition peut être utilisée comme après-shampoing, en particulier sur des cheveux sensibilisés. Cet après-shampooing peut être rincé ou non rincé et de préférence rincé.

Les compositions cosmétiques selon l'invention peuvent se présenter sous forme de gel, de lait, de crème, d'émulsion, de lotions fluides ou épaissies ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

La présente invention concerne également un procédé de traitement cosmétique des matières kératiniques telles que par exemple la peau ou les cheveux qui consiste à appliquer une quantité efficace d'une composition cosmétique telle que décrite ci-dessus, sur les matières kératiniques, à effectuer un éventuel rinçage après un éventuel temps de pose.

Le rinçage s'effectue par exemple avec de l'eau.

Ainsi, ce procédé selon l'invention permet le traitement, le conditionnement, le soin des cheveux ou de toute autre matière kératinique.

Les exemples suivants illustrent la présente invention et ne doivent être considérés en aucune manière comme limitant l'invention.

### EXEMPLES 1 A 3

On a préparé les compositions suivantes d'après-shampooing à rincer :

| | 1 | 2 | 3 |
|---|---|---|---|
| Alcool stéarylique | 2 g | 2 g | - |
| Alcool myristique | - | - | 2 g |
| N-oléoyl dihydrosphingosine | 0,4 g | 0,4 g | 0,4 g |
| Chlorure de béhényl triméthyl ammonium en solution aqueuse à 80% de MA (GENAMIN KDMP de CLARIANT) | 0,5 g (0,4 gMA) | 0,5 g (0,4 gMA) | - |
| Quaternium-87 (VARISOFT W575PG de GOLDSCHMIDT) | 3,35 g (2,5 gMA) | 3,35 g (2,5 gMA) | 3,35 g (2,5 gMA) |
| Chlorure de cétyl triméthyl ammonium en solution aqueuse à 25% de matière active (ARQUAD 16-25 LO d'AKZO NOBEL) | - | - | 1 g (0,25 gMA) |
| Poly(chlorure de diméthyldiallyl-ammonium) en solution aqueuse à 40% de matière active Densité de charge = 6,2 mEq/ (Merquat® 100 de ONDEO-NALCO) | - | 0,63 g (0,252 gMA) | - |
| Polycondensat de tétraméthyl hexaméthylènediamine en solution aqueuse à 60% MA (MEXOMER PO de CHIMEX) | 0,42 g (0,252 gMA) | 0,42 g (0,252 gMA) | 0,42 g (0,252 gMA) |
| Polydiméthylsiloxane à groupements aminoéthyl iminopropyl en émulsion aqueuse non ionique à 15% de MA (BELSIL ADM 8020 P de WACKER) | 5,9 g (0,885 gMA) | - | - |
| Polydiméthylsiloxane (DC200 FLUID - 60.000cs de DOW CORNING) | - | - | 1 g |
| Phosphate de di-amidon de maïs hydroxypropylé et prégélatinisé (STRUCTURE ZEA de NATIONAL STARCH) | 5 g | | 5 g |
| Fécule de pomme de terre modifiée par l'acide 2-chloroéthyl aminodipropionique neutralisée par la soude (Structure Solanace de NATIONAL STARCH) | - | 1 g | - |
| Parfum, conservateurs | qs | qs | qs |
| Eau qsp | 100 g | 100 g | 100 g |

Ces compositions ont été appliquées sur des cheveux très sensibilisés. Les propriétés cosmétiques (démêlage, lissage, souplesse) sont excellentes et homogènes des racines aux pointes des cheveux. Les pointes ne sont pas fourchues.

Entre deux applications, les cheveux restent doux, souples et lisses.

## Revendications

1. Composition cosmétique, comprenant, dans un milieu cosmétiquement acceptable, au moins un tensioactif cationique, au moins un amidon, au moins un polymère cationique non siliconé présentant une densité de charge cationique supérieure ou égale à 5 meq/g et au moins un composé solide non polymérique non ionique de point de fusion supérieur ou égal à 35°C et/ou ayant une viscosité à la température de 40°C et sous un taux de cisaillement de 1s⁻¹, supérieure ou égale à 1 Pa.s.

2. Composition selon la revendication précédente, **caractérisée en ce que** les tensioactifs cationiques sont choisis parmi les sels d'amines grasses primaires, secondaires ou tertiaires, éventuellement polyoxyalkylénées, les sels d'ammonium quaternaire et leurs mélanges.

3. Composition selon la revendication 2, **caractérisée en ce que** les sels d'ammonium quaternaire sont choisis parmi :
- ceux qui présentent la formule générale (V) suivante : 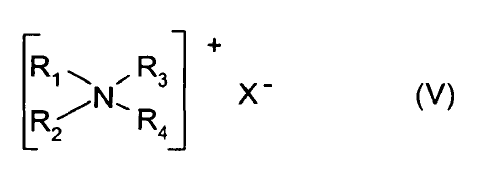 dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline ;
- les sels de diammonium quaternaire de formule (VII) : 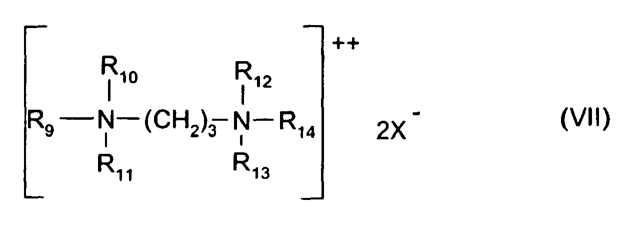 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X- est un anion choisi dans le groupe des halogénures, acétates, phosphates, nitrates et méthylsulfates ;
- les sels d'ammonium quaternaire comprenant au moins une fonction ester.

4. Composition selon la revendication 3, **caractérisée en ce que** les sels d'ammonium quaternaire de l'imidazoline sont choisis parmi ceux de formule (VI) suivante : 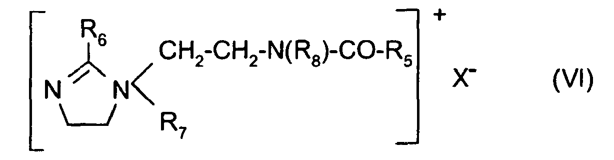 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène.

5. Composition selon l'une quelconque des revendications précédentes, **caractérisée en ce que** les tensioactifs cationiques sont choisis parmi le chlorure de béhényltriméthylammonium, le chlorure de cétyltriméthylammonium, le Quaternium-83, le Quaternium-87, le chlorure de béhénylamidopropyl 2,3-dihydroxypropyl diméthyl ammonium et le chlorure de palmitylamidopropyltriméthylammonium.

6. Composition selon l'une quelconque des revendications précédentes, **caractérisée en ce que** les tensioactifs cationiques sont contenus en une quantité allant de 0,05 à 10 % en poids, de préférence de 0,1 à 8 % en poids par rapport au poids total de la composition.

7. Composition cosmétique selon l'une quelconque des revendications précédentes, **caractérisée en ce que** le polymère cationique présente une densité de charge cationique allant de 5 à 20 méq/g.

8. Composition selon l'une quelconque des revendications précédentes, **caractérisée en ce que** le polymère cationique est choisi parmi les homopolymères et copolymères d'halogénure de dialkyldiallylammonium, les polyéthylène-imines, les polycondensats à motifs récurrents diammonium ou polyammonium quaternaire.

9. Composition selon l'une quelconque des revendications précédentes, **caractérisée en ce que** le polymère cationique est présent à une concentration allant de 0,01 % à 10 % en poids par rapport au poids total de la composition, de préférence de 0,05 % à 5 % en poids.

10. Composition selon l'une quelconque des revendications précédentes, **caractérisée par le fait que** les amidons sont choisis parmi les amidons de maïs, de riz, de manioc, d'orge, de pomme de terre, de blé, de sorgho, de pois.

11. Composition selon l'une quelconque des revendications précédentes, **caractérisée par le fait que** l'amidon est modifié par voie chimique et/ou physique.

12. Composition selon l'une quelconque des revendications précédentes, **caractérisée par le fait que** l'amidon est modifié par une ou plusieurs des réactions suivantes : prégélatinisation, oxydation, réticulation, estérification, étherification, amidification, traitements thermiques.

13. Composition selon l'une quelconque des revendications précédentes, **caractérisée par le fait que** l'amidon est modifié par au moins une estérification.

14. Composition selon l'une quelconque des revendications précédentes, **caractérisée par le fait que** l'amidon est choisi parmi les phosphates d'amidon.

15. Composition selon l'une quelconque des revendications précédentes, **caractérisée par le fait que** l'amidon est choisi parmi les amidons amphotères.

16. Composition selon la revendication précédente, **caractérisée en ce que** l'amidon amphotère est un chloroéthylamidodipropionate d'amidon.

17. Composition selon l'une quelconque des revendications précédentes, **caractérisée par le fait que** l'amidon est présent dans les composition de l'invention à une concentration allant de 0,01 à 10% en poids, de préférence de 0,05 à 5% par rapport au poids total de la composition.

18. Composition selon l'une quelconque des revendications précédentes, **caractérisée en ce que** le composé solide est choisi parmi les alcools gras oxyéthyléné ou non, les esters gras, les cires minérales, les cires organiques différentes des esters gras et des alcools gras, et leurs mélanges.

19. Composition selon l'une quelconque des revendications précédentes, **caractérisée en ce que** le composé solide est une cire végétale ou animales.

20. Composition selon l'une quelconque des revendications précédentes, **caractérisée en ce que** le composé solide est un alcool gras linéaire.

21. Composition selon l'une quelconque des revendications précédentes, **caractérisée en ce que** le composé solide est un alcool gras saturé.

22. Composition selon l'une quelconque des revendications précédentes, **caractérisé en ce que** le composé solide est un alcool gras présentant la structure R-OH, dans laquelle R désigne un radical alkyle comportant de 12 à 24 atomes de carbone éventuellement hydroxylé.

23. Composition selon l'une quelconque des revendications précédentes, **caractérisé en ce que** le composé solide est un alcool gras choisi parmi l'alcool myristique, l'alcool cétylique, l'alcool béhénylique, l'alcool stéarylique, et leurs mélanges.

24. Composition selon la revendication 18, **caractérisée en que** les esters gras sont des esters d'un acide carboxylique comportant au moins 10 atomes de carbone et d'un monoalcool ou d'un polyol.

25. Composition selon la revendication 24, **caractérisée en ce que** les esters gras sont choisis parmi des esters d'acide gras monocarboxylique comportant au moins 10 atomes de carbone et d'un monoalcool comportant au moins 10 atomes de carbone.

26. Composition selon la revendication 25, **caractérisée en ce que** les esters gras sont choisis parmi le myristate de cétyle, le myristate de myristyle.

27. Composition selon l'une quelconque des revendications 18 ou 19, **caractérisée en ce que** les cires sont choisies parmi la cire d'abeille, le spermaceti, la cire de lanoline et les dérivés de lanoline ; la cire de Carnauba, de Candellila, d'Ouricury, du Japon, le beurre de cacao ou les cires de fibres de liège ou de canne à sucre ; les cires de paraffine, de vaseline, de lignite ou les cires microcristallines, les ozokérite, la cire d'olivier, la cire de riz, la cire de jojoba hydrogénée ou les cires absolues de fleurs, les cires d'abeilles, ou les cires d'abeilles modifiées (cerabellina), les céramides naturelles ou synthétiques et leurs mélanges.

28. Composition selon l'une quelconque des revendications précédentes, **caractérisée par le fait que** le composé solide est présent dans les composition de l'invention à une concentration comprise entre 0,1 et 15% en poids, de préférence entre 0,5 et 10% par rapport au poids total de la composition.

29. Composition selon l'une quelconque des revendications précédentes, **caractérisée en ce qu'**elle comprend en outre au moins un agent conditionneur additionnel.

30. Composition selon la revendication 29, **caractérisée en ce que** l'agent conditionneur additionnel est choisi parmi les silicones, les polymères cationiques différents des polymères cationiques selon l'invention, les esters carboxyliques liquides, les huiles végétales, les huiles minérales, les huiles de synthèse et leurs mélanges.

31. Composition selon l'une quelconque des revendications précédentes, **caractérisée en ce que** le milieu aqueux cosmétiquement acceptable est constitué par de l'eau ou par un mélange d'eau et d'un solvant cosmétiquement acceptable.

32. Composition selon la revendication 31, **caractérisée en ce que** le solvant cosmétiquement acceptable est choisi parmi les alcools inférieur en C₁-C₄ tel que l'éthanol, l'isopropanol, le tertio-butanol, le n-butanol ; les alkylèneglycols comme le propylèneglycol, les éthers de polyol ; les alcanes en C₅-C₁₀ et leurs mélanges.

33. Composition selon l'une quelconque des revendications précédentes, **caractérisée en ce qu'**elle comprend en outre des additifs tels que des polymères anioniques, non ioniques ou amphotères, des épaississants comme des acides ou des électrolytes, des opacifiants, des agents nacrants, des vitamines, des provitamines telles que le panthénol, des parfums, des colorants, des particules organiques ou minérales, des conservateurs, des agents de stabilisation du pH.

34. Composition selon l'une quelconque des revendications précédentes, **caractérisée par le fait qu'**elle se présente sous forme d'après-shampooing, de composition pour la permanente, le défrisage, la coloration ou la décoloration des cheveux, de composition à rincer à appliquer entre les deux étapes d'une permanente ou d'un défrisage.

35. Composition selon l'une quelconque des revendications précédentes, **caractérisée en ce qu'**elle est se présente sous la forme d'un après-shampoing à rincer.

36. Procédé de traitement cosmétique des matières kératiniques, telles que les cheveux, **caractérisé en ce qu'**il consiste à appliquer sur lesdites matières une quantité efficace d'une composition cosmétique selon l'une des revendications 1 à 35, puis à effectuer éventuellement un rinçage.

## Claims

1. Cosmetic composition comprising, in a cosmetically acceptable medium, at least one cationic surfactant, at least one starch, at least one non-silicone cationic polymer with a cationic charge density of greater than or equal to 5 meq./g and at least one nonionic non-polymeric solid compound with a melting point of greater than or equal to 35°C and/or with a viscosity, at a temperature of 40°C and at a shear rate of 1 s⁻¹, of greater than or equal to 1 Pa.s.

2. Composition according to the preceding claim, **characterized in that** the cationic surfactants are chosen from optionally polyoxyalkylenated primary, secondary or tertiary fatty amine salts and quaternary ammonium salts, and mixtures thereof.

3. Composition according to Claim 2, **characterized in that** the quaternary ammonium salts are chosen from:
- those having the general formula (V) below: 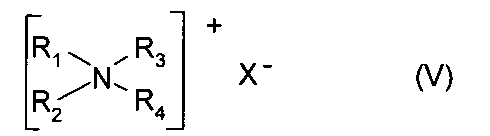 in which the symbols R₁ to R₄, which may be identical or different, represent a linear or branched aliphatic radical containing from 1 to 30 carbon atoms or an aromatic radical such as aryl or alkylaryl; X⁻ is an anion chosen from the group of halides, phosphates, acetates, lactates, (C₂-C₆) alkyl sulfates, alkylsulfonates and alkylarylsulfonates;
- quaternary ammonium salts of imidazoline;
- diquaternary ammonium salts of formula (VII): 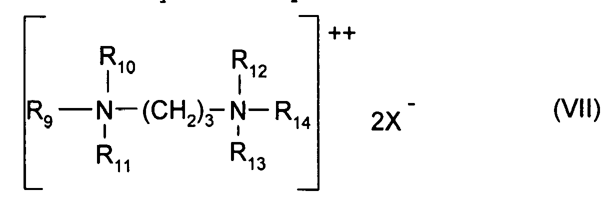 in which R₉ denotes an aliphatic radical containing from about 16 to 30 carbon atoms, R₁₀, R₁₁, R₁₂, R₁₃ and R₁₄, which may be identical or different, are chosen from hydrogen or an alkyl radical containing from 1 to 4 carbon atoms, and X⁻ is an anion chosen from the group of halides, acetates, phosphates, nitrates and methyl sulfates;
- quaternary ammonium salts comprising at least one ester function.

4. Composition according to Claim 3, **characterized in that** the quaternary ammonium salts of imidazoline are chosen from those of formula (VI) below: 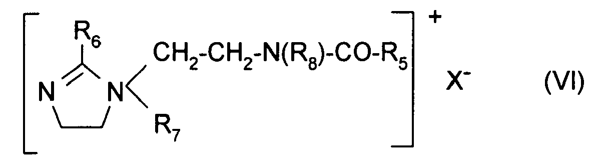 in which R₅ represents an alkenyl or alkyl radical containing from 8 to 30 carbon atoms, for example tallow fatty acid derivatives, R₆ represents a hydrogen atom, a C₁-C₄ alkyl radical or an alkenyl or alkyl radical containing from 8 to 30 carbon atoms, R₇ represents a C₁-C₄ alkyl radical, R₈ represents a hydrogen atom or a C₁-C₄ alkyl radical, X⁻ is an anion chosen from the group of halides, phosphates, acetates, lactates, alkyl sulfates, alkylsulfonates and alkylarylsulfonates. Preferably, R₅ and R₆ denote a mixture of alkenyl or alkyl radicals containing from 12 to 21 carbon atoms, for example tallow fatty acid derivatives, R₇ denotes methyl and R₈ denotes hydrogen.

5. Composition according to any one of the preceding claims, **characterized in that** the cationic surfactants are chosen from behenyltrimethylammonium chloride, cetyltrimethylammonium chloride, Quaternium-83, Quaternium-87, behenylamidopropyl-2,3-dihydroxypropyldimethylammonium chloride and palmitylamidopropyltrimethylammonium chloride.

6. Composition according to any one of the preceding claims, **characterized in that** the cationic surfactants are contained in an amount ranging from 0.05% to 10% by weight and preferably from 0.1% to 8% by weight relative to the total weight of the composition.

7. Cosmetic composition according to any one of the preceding claims, **characterized in that** the cationic polymer has a cationic charge density ranging from 5 to 20 meq./g.

8. Composition according to any one of the preceding claims, **characterized in that** the cationic polymer is chosen from dialkyldiallylammonium halide homopolymers and copolymers, polyethyleneimines and polycondensates containing diquaternary ammonium or polyquaternary ammonium repeating units.

9. Composition according to any one of the preceding claims, **characterized in that** the cationic polymer is present in a concentration ranging from 0.01% to 10% by weight and preferably from 0.05% to 5% by weight relative to the total weight of the composition.

10. Composition according to any one of the preceding claims, **characterized in that** the starches are chosen from corn starch, rice starch, cassava starch, barley starch, potato starch, wheat starch, sorghum starch and pea starch.

11. Composition according to any one of the preceding claims, **characterized in that** the starch is chemically and/or physically modified.

12. Composition according to any one of the preceding claims, **characterized in that** the starch is modified via one or more of the following reactions: pregelatinization, oxidation, crosslinking, esterification, etherification, amidation, heat treatments.

13. Composition according to any one of the preceding claims, **characterized in that** the starch is modified via at least one esterification.

14. Composition according to any one of the preceding claims, **characterized in that** the starch is chosen from starch phosphates.

15. Composition according to any one of the preceding claims, **characterized in that** the starch is chosen from amphoteric starches.

16. Composition according to the preceding claim, **characterized in that** the amphoteric starch is a starch chloroethylamidodipropionate.

17. Composition according to any one of the preceding claims, **characterized in that** the starch is present in the composition of the invention in a concentration ranging from 0.01% to 10% and preferably from 0.05% to 5% by weight relative to the total weight of the composition.

18. Composition according to any one of the preceding claims, **characterized in that** the solid compound is chosen from oxyethylenated or non-oxyethylenated fatty alcohols, fatty esters, mineral waxes, organic waxes other than fatty esters and fatty alcohols, and mixtures thereof.

19. Composition according to any one of the preceding claims, **characterized in that** the solid compound is a plant or animal wax.

20. Composition according to any one of the preceding claims, **characterized in that** the solid compound is a linear fatty alcohol.

21. Composition according to any one of the preceding claims, **characterized in that** the solid compound is a saturated fatty alcohol.

22. Composition according to any one of the preceding claims, **characterized in that** the solid compound is a fatty alcohol having the structure R-OH, in which R denotes an optionally hydroxylated alkyl radical containing from 12 to 24 carbon atoms.

23. Composition according to any one of the preceding claims, **characterized in that** the solid compound is a fatty alcohol chosen from myristyl alcohol, cetyl alcohol, behenyl alcohol and stearyl alcohol, and mixtures thereof.

24. Composition according to Claim 18, **characterized in that** the fatty esters are esters of a carboxylic acid containing at least 10 carbon atoms and of a monoalcohol or a polyol.

25. Composition according to Claim 24, **characterized in that** the fatty esters are chosen from esters of a monocarboxylic fatty acid containing at least 10 carbon atoms and of a monoalcohol containing at least 10 carbon atoms.

26. Composition according to Claim 25, **characterized in that** the fatty esters are chosen from cetyl myristate and myristyl myristate.

27. Composition according to either of Claims 18 and 19, **characterized in that** the waxes are chosen from beeswax, spermaceti, lanolin wax and lanolin derivatives; carnauba wax, candelilla wax, ouricury wax, Japan wax, cocoa butter, cork fibre wax or sugarcane wax; paraffin wax, petroleum jelly wax, lignite wax or microcrystalline waxes, ozokerites, olive wax, rice wax, hydrogenated jojoba wax or the absolute waxes of flowers, beeswaxes or modified beeswaxes (cerabellina), and natural or synthetic ceramides, and mixtures thereof.

28. Composition according to any one of the preceding claims, **characterized in that** the solid compound is present in the composition of the invention in a concentration of between 0.1% and 15% and preferably between 0.5% and 10% by weight relative to the total weight of the composition.

29. Composition according to any one of the preceding claims, **characterized in that** it also comprises at least one additional conditioning agent.

30. Composition according to Claim 29, **characterized in that** the additional conditioning agent is chosen from silicones, cationic polymers other than the cationic polymers according to the invention, liquid carboxylic esters, plant oils, mineral oils and synthetic oils, and mixtures thereof.

31. Composition according to any one of the preceding claims, **characterized in that** the cosmetically acceptable aqueous medium consists of water or of a mixture of water and a cosmetically acceptable solvent.

32. Composition according to Claim 31, **characterized in that** the cosmetically acceptable solvent is chosen from C₁-C₄ lower alcohols such as ethanol, isopropanol, tert-butanol or n-butanol; alkylene glycols, for instance propylene glycol or polyol ethers; C₅-C₁₀ alkanes, and mixtures thereof.

33. Composition according to any one of the preceding claims, **characterized in that** it also comprises additives such as anionic, nonionic or amphoteric polymers, thickeners, for instance acids or electrolytes, opacifiers, nacreous agents, vitamins, provitamins such as panthenol, fragrances, dyes, organic or mineral particles, preserving agents and pH stabilizers.

34. Composition according to any one of the preceding claims, **characterized in that** it is in the form of a hair conditioner, a composition for permanent-waving, relaxing, dyeing or bleaching the hair, or a rinse-out composition to be applied between the two steps of a permanent-waving or hair-relaxing operation.

35. Composition according to any one of the preceding claims, **characterized in that** it is in the form of a rinse-out hair conditioner.

36. Cosmetic process for treating keratin materials, such as the hair, **characterized in that** it consists in applying to the said materials an effective amount of a cosmetic composition according to one of Claims 1 to 35, and then in optionally rinsing.

## Patentansprüche

1. Kosmetische Zusammensetzung, die in einem kosmetisch akzeptablen Medium mindestens einen kationischen grenzflächenaktiven Stoff, mindestens eine Stärke, mindestens ein nicht siliconiertes kationisches Polymer, das eine kationische Ladungsdichte von 5 meq/g oder darüber aufweist, und mindestens eine nichtionische, nicht polymere Verbindung mit einem Schmelzpunkt von 35 °C oder darüber und/oder einer Viskosität von 1 Pa.s oder darüber bei einer Temperatur von 40 °C und einem Schergrad von 1 s⁻¹ enthält.

2. Zusammensetzung nach dem vorhergehenden Anspruch, **dadurch gekennzeichnet, dass** die kationischen grenzflächenaktiven Stoffe unter den Salzen von primären, sekundären oder tertiären Fettaminen, die gegebenenfalls polyalkoxyliert sind, quartären Ammoniumsalzen und deren Gemischen ausgewählt sind.

3. Zusammensetzung nach Anspruch 2, **dadurch gekennzeichnet, dass** die quartären Ammoniumsalze ausgewählt sind unter:
- quartären Ammoniumsalzen der folgenden allgemeinen Formel (V): 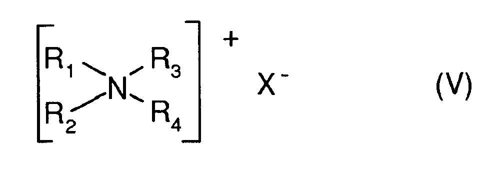 worin die Gruppen R₁ bis R₄, die gleich oder verschieden sein können, eine geradkettige oder verzweigte, aliphatische Gruppe mit 1 bis 30 Kohlenstoffatomen oder eine aromatische Gruppe, wie Aryl oder Alkylaryl, bedeuten; und X⁻ ein Anion ist, das unter den Halogeniden, Phosphaten, Acetaten, Lactaten, Alkyl(C₂₋₆)sulfaten, Alkyl- oder Alkylarylsulfonaten ausgewählt ist;
- quartären Ammoniumsalzen von Imidazolin;
- quartären Diammoniumsalzen der Formel (VII): 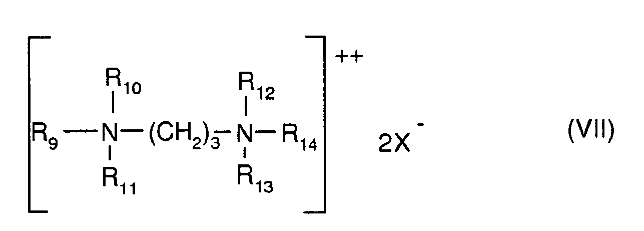 worin die Gruppe R₉ eine aliphatische Gruppe mit etwa 16 bis 30 Kohlenstoffatomen bedeutet, die Gruppen R₁₀, R₁₁, R₁₂, R₁₃ und R₁₄, die gleich oder verschieden sind, unter Wasserstoff oder einer Alkylgruppe mit 1 bis 4 Kohlenstoffatomen ausgewählt sind, und X⁻ ein Anion ist, das unter den Halogeniden, Acetaten, Phosphaten, Nitraten und Methylsulfaten ausgewählt ist;
- quartären Ammoniumsalzen, die mindestens eine Esterfunktion enthalten.

4. Zusammensetzung nach Anspruch 3, **dadurch gekennzeichnet, dass** die quartären Ammoniumsalze von Imidazolin unter den Verbindungen der folgenden Formel (VI) ausgewählt sind: 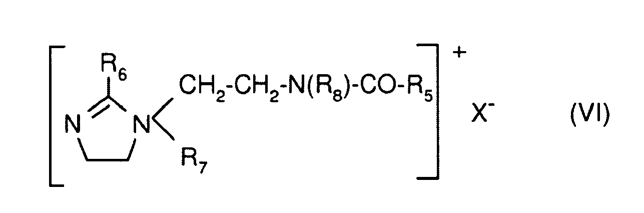 worin R₅ eine Alkenyl- oder Alkylgruppe mit 8 bis 30 Kohlenstoffatomen bedeutet, die beispielsweise von Fettsäuren aus Talg abgeleitet sind, R₆ ein Wasserstoffatom, C₁₋₄-Alkyl oder eine Alkenyl- oder Alkylgruppe mit 8 bis 30 Kohlenstoffatomen bedeutet, R₇ eine C₁₋₄-Alkylgruppe ist, R₈ ein Wasserstoffatom oder C₁₋₄-Alkyl bedeutet und X ein Anion ist, das unter den Halogeniden, Phosphaten, Acetaten, Lactaten, Alkylsulfaten, Alkyl- oder Alkylarylsulfonaten ausgewählt ist; vorzugsweise bedeuten die Gruppen R₅ und R₆ ein Gemisch aus Alkenyl- oder Alkylgruppen mit 12 bis 21 Kohlenstoffatomen, die beispielsweise von Talgfettsäuren abgeleitet sind, die Gruppe R₇ Methyl und die Gruppe R₈ Wasserstoff.

5. Zusammensetzung nach einem der vorhergehenden Ansprüche, **dadurch gekennzeichnet, dass** die kationischen grenzflächenaktiven Stoffe unter Behenyltrimethylammoniumchlorid, Cetyltrimethylammoniumchlorid, Quaternium-83, Quaternium-87, Behenylamidopropyl-2,3-dihydroxypropyldimethylammoniumchlorid und Palmitylamidopropyltrimethylammoniumchlorid ausgewählt sind.

6. Zusammensetzung nach einem der vorhergehenden Ansprüche, **dadurch gekennzeichnet, dass** die grenzflächenaktiven Stoffe in einem Mengenanteil von 0,05 bis 10 Gew.-% und vorzugsweise im Bereich von 0,1 bis 8 Gew.-%, bezogen auf das Gesamtgewicht der Zusammensetzung, enthalten sind.

7. Kosmetische Zusammensetzung nach einem der vorhergehenden Ansprüche, **dadurch gekennzeichnet, dass** das kationische Polymer eine kationische Ladungsdichte von 5 bis 20 meq/g aufweist.

8. Zusammensetzung nach einem der vorhergehenden Ansprüche, **dadurch gekennzeichnet, dass** das kationische Polymer unter den Homopolymeren und Copolymeren von Dialkyldiallylammoniumhalogeniden, Polyethyleniminen, Polykondensaten mit wiederkehrenden quartären Diammonium- oder Polyammoniumeinheiten ausgewählt ist.

9. Zusammensetzung nach einem der vorhergehenden Ansprüche, **dadurch gekennzeichnet, dass** das kationische Polymer in einer Konzentration von 0,01 bis 10 Gew.-%, bezogen auf das Gesamtgewicht der Zusammensetzung, und vorzugsweise im Bereich von 0,05 bis 5 Gew.-% enthalten ist.

10. Zusammensetzung nach einem der vorhergehenden Ansprüche, **dadurch gekennzeichnet, dass** die Stärkeverbindungen unter den Stärken aus Mais, Reis, Maniok, Gerste, Kartoffel, Weizen, Sorgho und Erbse ausgewählt sind.

11. Zusammensetzung nach einem der vorhergehenden Ansprüche, **dadurch gekennzeichnet, dass** die Stärke auf chemischem und/oder physikalischen Weg modifiziert wurde.

12. Zusammensetzung nach dem vorhergehenden Anspruch, **dadurch gekennzeichnet, dass** die Stärke durch eine oder mehrere der folgenden Reaktionen modifiziert ist: Vorverkleisterung, Oxidation, Vernetzung, Veresterung, Veretherung, Amidierung, thermische Behandlungen.

13. Zusammensetzung nach einem der vorhergehenden Ansprüche, **dadurch gekennzeichnet, dass** die Stärke durch mindestens eine Veresterung modifiziert ist.

14. Zusammensetzung nach einem der vorhergehenden Ansprüche, **dadurch gekennzeichnet, dass** die Stärke unter den Stärkephosphaten ausgewählt ist.

15. Zusammensetzung nach einem der vorhergehenden Ansprüche, **dadurch gekennzeichnet, dass** die Stärke unter den amphoteren Stärken ausgewählt ist.

16. Zusammensetzung nach dem vorhergehenden Anspruch,
**dadurch gekennzeichnet, dass** die amphotere Stärke ein Stärkechlorethylamidodipropionat ist.

17. Zusammensetzung nach einem der vorhergehenden Ansprüche, **dadurch gekennzeichnet, dass** die Stärke in den erfindungsgemäßen Zusammensetzungen in einer Konzentration von 0,01 bis 10 Gew.-% und vorzugsweise im Bereich von 0,05 bis 5 Gew.-% des Gesamtgewichts der Zusammensetzung enthalten ist.

18. Zusammensetzung nach einem der vorhergehenden Ansprüche, **dadurch gekennzeichnet, dass** die feste Verbindung unter den Fettalkoholen, die polyethoxyliert oder nicht polyethoxyliert sind, Fettsäuerestern, Mineralwachsen, organischen Wachsen, die von den Fettsäureestern und Fettalkoholen verschieden sind, und deren Gemischen ausgewählt ist.

19. Zusammensetzung nach einem der vorhergehenden Ansprüche, **dadurch gekennzeichnet, dass** die feste Verbindung ein pflanzliches oder tierisches Wachs ist.

20. Zusammensetzung nach einem der vorhergehenden Ansprüche, **dadurch gekennzeichnet, dass** die feste Verbindung ein linearer Fettalkohol ist.

21. Zusammensetzung nach einem der vorhergehenden Ansprüche, **dadurch gekennzeichnet, dass** die feste Verbindung ein gesättigter Fettalkohol ist.

22. Zusammensetzung nach einem der vorhergehenden Ansprüche, **dadurch gekennzeichnet, dass** die feste Verbindung ein Fettalkohol ist, der die Struktur R-OH aufweist, wobei R eine Alkylgruppe mit 12 bis 24 Kohlenstoffatomen bedeutet, die gegebenenfalls hydroxyliert ist.

23. Zusammensetzung nach einem der vorhergehenden Ansprüche, **dadurch gekennzeichnet, dass** die feste Verbindung ein Fettalkohol ist, der unter Myristylalkohol, Cetylalkohol, Behenylalkohol, Stearylalkohol und deren Gemischen ausgewählt ist.

24. Zusammensetzung nach Anspruch 18, **dadurch gekennzeichnet, dass** die Fettsäureester Ester einer Carbonsäure mit mindestens 10 Kohlenstoffatomen und eines Monoalkohols oder eines Polyols sind.

25. Zusammensetzung nach Anspruch 24, **dadurch gekennzeichnet, dass** die Fettsäureester unter den Ester einer Monocarbonsäure mit mindestens 10 Kohlenstoffatomen und eines Monoalkohols mit mindestens 10 Kohlenstoffatomen ausgewählt sind.

26. Zusammensetzung nach Anspruch 25, **dadurch gekennzeichnet, dass** die Fettsäureester unter Cetylmyristat und Myristylmyristat ausgewählt sind.

27. Zusammensetzung nach einem der Ansprüche 18 oder 19,
**dadurch gekennzeichnet, dass** die Wachse unter Bienenwachs, Spermaceti, Lanolinwachs und den Lanolinderivaten; Carnaubawachs, Candelillawachs, Ouricurywachs, Japanwachs, Kakaobutter, Korkfaserwachs oder Zuckerrohrwachs; Paraffinwachs, Vaseline, Lignit oder mikrokristallinen Wachsen, Ozokerit, Ölbaumwachs, Reiswachs, hydriertem Jojobawachs oder reinen Blütenwachsen, Bienenwachsen oder modifizierten Bienenwachsen (Cera bellina), natürlichen oder synthetischen Ceramiden und deren Gemischen ausgewählt sind.

28. Zusammensetzung nach einem der vorhergehenden Ansprüche, **dadurch gekennzeichnet, dass** die feste Verbindung in der erfindungsgemäßen Zusammensetzung in einer Konzentration von 0,1 bis 15 Gew.-% und vorzugsweise im Bereich von 0,5 bis 10 %, bezogen auf das Gesamtgewicht der Zusammensetzung, enthalten ist.

29. Zusammensetzung nach einem der vorhergehenden Ansprüche, **dadurch gekennzeichnet, dass** sie ferner mindestens ein ergänzendes Konditioniermittel enthält.

30. Zusammensetzung nach Anspruch 29, **dadurch gekennzeichnet, dass** das ergänzende Konditioniermittel unter den Siliconen, kationischen Polymeren, die von den erfindungsgemäßen kationischen Polymeren verschieden sind, flüssigen Carbonsäureestern, pflanzlichen Ölen, Mineralölen, synthetischen Ölen und deren Gemischen ausgewählt ist.

31. Zusammensetzung nach einem der vorhergehenden Ansprüche, **dadurch gekennzeichnet, dass** das kosmetisch akzeptable wässrige Medium aus Wasser oder aus einem Gemisch von Wasser und einem kosmetisch akzeptablen Lösungsmittel besteht.

32. Zusammensetzung nach Anspruch 31, **dadurch gekennzeichnet, dass** das kosmetisch akzeptable Lösemittel unter den niederen C₁₋₄-Alkoholen, wie Ethanol, Isopropanol, tert-Butanol, n-Butanol; Alkylenglycolen, wie Propylenglycol, Polyolethern; C₅₋₁₀-Alkanen und deren Gemischen ausgewählt ist.

33. Zusammensetzung nach einem der vorhergehenden Ansprüche, **dadurch gekennzeichnet, dass** sie ferner Additive enthält, wie beispielsweise anionische, nichtionische oder amphotere Polymere, Verdickungsmittel, wie Säuren oder Elektrolyte, Trübungsmittel, Perlglanzstoffe, Vitamine, Provitamine, wie Panthenol, Parfums, Farbmittel, organische oder anorganische Partikel, Konservierungsmittel, pH-Stabilisatoren.

34. Zusammensetzung nach einem der vorhergehenden Ansprüche, **dadurch gekennzeichnet, dass** sie in Form einer Pflegespülung, als Zusammensetzung für dauerhafte Verformungen, zum Entkräuseln, zum Färben oder zum Entfärben der Haare, als Zusammensetzung, die ausgespült wird und zwischen den beiden Schritten einer dauerhaften Verformung oder einer Entkräuselung aufgebracht wird, vorliegt.

35. Zusammensetzung nach einem der vorhergehenden Ansprüche, **dadurch gekennzeichnet, dass** sie in Form einer Pflegespülung vorliegt, die ausgespült wird.

36. Verfahren zur kosmetischen Behandlung von Keratinsubstanzen, wie Haaren, **dadurch gekennzeichnet, dass** es darin besteht, auf die Keratinsubstanzen eine wirksame Menge einer kosmetischen Zusammensetzung nach einem der Ansprüche 1 bis 35 aufzubringen und anschließend gegebenenfalls zu spülen.
